# EUROPÄISCHE PATENTSCHRIFT

(11) **EP 0 923 355 B1**
(45) Veröffentlichungstag und Bekanntmachung des Hinweises auf die Patenterteilung: **22.01.2003**
(21) Anmeldenummer: 97924915.8
(22) Anmeldetag: 22.05.1997
(51) Int. Cl.: A61F 2/44

(54) **IMPLANTAT ZUM FUSIONIEREN VON ZWEI BENACHBARTEN WIRBELN DER WIRBELSÄULE**
IMPLANT FOR BONDING TWO ADJACENT VERTEBRAE OF THE VERTEBRAL COLUMN
IMPLANT POUR FUSIONNER DEUX VERTEBRES ADJACENTES DE LA COLONNE VERTEBRALE

(30) Priorität: 26.07.1996 DE 19630256
(43) Veröffentlichungstag der Anmeldung: 23.06.1999
(73) Patentinhaber: ULRICH, Heinrich, 89075 Ulm/Donau (DE)
(72) Erfinder: ULRICH, Heinrich, D-89075 Ulm/Donau (DE); SCHÖNHÖFFER, Helmut, D-89077 Ulm (DE)
(74) Vertreter: Dziewior, Joachim
(86) Internationale Anmeldenummer: DE9701067
(87) Internationale Veröffentlichungsnummer: WO98004217

(56) Entgegenhaltungen:
- EP-A- 0 307 241
- WO-A-87/07827
- WO-A-95/26164
- DE-A- 4 302 397
- DE-U- 29 511 146
- FR-A- 2 710 519
- FR-A- 2 727 005
- US-A- 4 501 269
- US-A- 5 092 893

## Beschreibung

Die Erfindung betrifft ein Implantat zum Fusionieren von zwei unmittelbar benachbarten Wirbeln der Wirbelsäule, mit einem Zapfen zum Einstecken in einen an den sich gegenüber liegenden beiden Flächen der Wirbelkörper als Zapfenaufnahme ausgebildeten Bohrkanal.

Implantate dieser Art sind aus US-A-4 501 269, EP-A-0 307 241 oder WO 87/07827 bekannt und dienen, im Bereich der Lendenwirbelsäule zumeist in einer paarweisen Anordnung parallel nebeneinander, zur Fusionierung von in der Wirbelsäule benachbarten Wirbeln nach teilweise oder vollständiger Entfernung der Bandscheibe. Die beiden Wirbelkörper werden an ihren einander gegenüber liegenden Flächen mit einander zugeordneten nutartigen Ausnehmungen versehen, die gemeinsam den Bohrkanal für die Aufnahme des Zapfens bilden, wobei der Bohrkanal - je nach Operationstechnik - in Richtung anterior oder posterior offen mündet und entsprechend posterior bzw. anterior in den Wirbelkörpern blind endet, also das Einführen des Zapfens in den Bohrkanal entsprechend von anterior bzw. posterior erfolgt, bis der Zapfen vollständig im Bohrkanal aufgenommen ist, das in Einsteckrichtung rückwärtige Ende des Zapfens also nicht mehr aus dem Bohrkanal vorsteht. Die Lage des Zapfens im Bohrkanal muß, insbesondere in Einsteckrichtung, aber auch gegen Verdrehen, möglichst fixiert sein, damit spätere Verschiebungen bzw. Verdrehungen des Zapfens gegenüber den Wirbeln und dadurch mögliche spätere Verlagerungen der Wirbel gegeneinander möglichst verhindert werden. Aus diesem Grund können die Zapfen an ihrer Umfangsfläche mit Vorsprüngen in verschiedenster Ausbildung versehen sein, die an der Bohrkanalwandung in die Wirbelkörper eindringen und dadurch die Zapfen an den Wirbelkörpern gleichsam verankern sollen. Dabei bestimmt allerdings die Tiefe des Eindringens den gegenseitigen Abstand der Wirbel in Höhenrichtung. Vergrößert sich daher diese Eindringtiefe durch die spätere Belastung der Wirbel nach der Implantation, nähern sich die Wirbel einander, was, ebenso wie die schon angesprochenen gegenseitigen Wirbelverschiebungen, zu neuen Schwierigkeiten führen und den Erfolg der Operation beeinträchtigen kann.

Der Erfindung liegt die Aufgabe zugrunde, ein Implantat der eingangs genannten Art so auszubilden, daß die Lage des Implantats relativ zu den Wirbeln und damit die Lage der Wirbel zueinander sicher fixiert ist, spätere Dislozierungen also nicht mehr auftreten können.

Diese Aufgabe wird nach der Erfindung durch die Merkmale des Anspruchs 1 gelöst.

Bei dem erfindungsgemäßen Implantat übernimmt die Platte die Fixierung des Zapfens gegenüber den Wirbeln; die mit den Knochenschrauben an den Wirbelkörpern befestigte Platte fixiert den Zapfen in Längsrichtung des Bohrkanals und sichert ihn gegen Austreten aus dem Bohrkanal. Weiter fixiert die Platte unmittelbar die durch sie verbundenen beiden Wirbel gegeneinander in Höhenrichtung, so daß sich die Wirbel auch in dieser Richtung nicht gegenseitig verlagern können, sollte sich an den Bohrkanalwänden ein tieferes Eindringen des Zapfens in die Wirbelkörper postoperativ ergeben. Im Ergebnis verhindert das erfindungsgemäße Implantat durch die den Zapfen haltende Platte ein gegenseitiges Dislozieren der beiden fusionierten Wirbel in zwei Richtungen, nämlich in Richtung des Bohrkanals wie auch quer dazu in Höhenrichtung, und schließlich kann die Platte auch den Zapfen gegen Verdrehen um die Zapfenachse sichern. Dabei setzt die Verwendung des Implantats selbstverständlich voraus, daß an den Wirbelkörpern der zu fusionierenden Wirbel geeignete Anlageflächen für die Platte vorhanden oder vor der Implantation ausgebildet worden sind; jedoch ist diese Voraussetzung jedenfalls dann im allgemeinen erfüllt, wenn die Implantation von vorn - anterior - erfolgt.

Die lösbare Halterung des Zapfens an der Platte erfolgt dadurch, daß zunächst nur der Zapfen - gelöst von der Platte - in den Bohrkanal eingesetzt und erst dann der Zapfen mit der Platte verbunden und die Platte an den benachbarten Wirbeln befestigt werden kann. Zur lösbaren Halterung des Zapfens an der Platte ist eine Verbindungsschraube vorgesehen, die in koaxiale Öffnungen des Zapfens und der Platte einsetzbar und dabei mit dem Schraubengewinde in ein an der Innenwand der Zapfenöffnung ausgebildetes Muttergewinde eindrehbar ist. Die Verbindungsschraube fixiert die Platte am Zapfen in Achsenrichtung der Schraube, läßt aber zunächst noch, bis zum abschließenden Anziehen der Verbindungsschraube, ein drehendes Ausrichten der Platte gegenüber dem schon im Bohrkanal positionierten Zapfen zu. Im einzelnen empfiehlt es sich, daß der Schraubenkopf der Verbindungsschraube in die Öffnung der Platte eingesenkt ist, wobei der Schraubenkopf mit einer kegelstumpfförmigen Ringfläche einer entsprechenden Ringfläche an der Innenwand der Plattenöffnung aufsitzt. Auch ist sehr zweckmäßig, daß sich die kegelstumpfförmige Ringfläche am Schraubenkopf über die gesamte axiale Höhe des Schraubenkopfes erstreckt. Die gegenseitige Verdrehungssicherheit wird dadurch erreicht, daß am Zapfen und an der Platte einander zugeordnete Verzahnungen ausgebildet sind, die bei an der Platte gehaltenem Zapfen miteinander zum Eingriff kommen und ein gegenseitiges Verdrehen von Zapfen und Platte um die Achse der Verbindungsschraube verhindern. Die Verzahnungen sind als die Öffnungen in der Platte bzw. im Zapfen umgebende Ringverzahnung mit radial zur Achse der Verbindungsschraube gerichteten Zahnflächen ausgebildet.

Das erfindungsgemäße Implantat kann im allgemeinen in jedem Bereich der Wirbelsäule, also Lendenwirbelsäule, Brustwirbelsäule oder Halswirbelsäule zum Einsatz kommen, und zwar jeweils einzeln oder, wo genügend Platz zur Verfügung steht, wie insbesondere im Bereich der Lendenwirbelsäule, jeweils paarweise. Dabei kann der Zapfen je nach den speziellen Erfordernissen unterschiedlich ausgebildet sein. Er kann eckigen oder runden, insbesondere kreisförmigen Umriß besitzen, zylindrisch sein oder sich in Richtung zur Platte hin beispielsweise kegelig oder pyramidenförmig erweitern; er kann massiv oder mit Hohlräumen versehen sein, um in letzterem Fall das Zapfeninnere mit Knochenzement oder vorzugsweise autologem Knochenmaterial füllen zu können, um das Einwachsen des umgebenden Knochengewebes zu ermöglichen. Insbesondere für die Verwendung im Halswirbelsäulenbereich empfiehlt sich im übrigen ein möglichst offen gestalteter Zapfen. Eine dafür besonders bevorzugte Ausführungsform ist dadurch gekennzeichnet, daß der Zapfen als überwiegend hohler Körper ausgebildet ist, der am plattenseitigen Zapfenende einen mit der Platte verbindbaren Stirnring und am anderen Zapfenende eine Stirnscheibe sowie mindestens einen, vorzugsweise zwei den Stirnring mit der Stirnscheibe verbindende Stege aufweist. Zweckmäßigerweise sind die Ebenen des Stirnrings und der Stirnscheibe senkrecht und die sie verbindenden Stege parallel zur Einsteckrichtung des Zapfens in den Bohrkanal ausgerichtet. Der Stirnring und die Stirnscheibe können kreisförmigen Umriß bei gleichem Kreisdurchmesser aufweisen. In weiter bevorzugter Ausführungsform ist der Stirnring an seiner inneren Umfangswandung mit dem Muttergewinde für die Verbindungsschraube versehen. Auch empfiehlt es sich, daß die Platte einen ihre Öffnung für die Verbindungsschraube umgebenden Kragen aufweist, an dem sich der Stirnring des Zapfens abstützt. Der Kragen und der Zapfen können in ihrem äußeren Umriß übereinstimmen und an ihren einander zugekehrten Ringflächen die Verzahnungen aufweisen. Um den Zapfen beim Einsetzen in den Bohrkanal mit einem Instrument gut handhaben zu können, empfiehlt es sich weiter, daß die Stirnscheibe eine Aufnahme zum Einsetzen des Instrumentes aufweist, mit dem der Zapfen bei zunächst noch fehlender Platte in den Bohrkanal eingesteckt und darin kontrolliert positioniert werden kann. Damit dabei das Instrument innen durch den Zapfen hindurchgeführt werden kann, muß der Steg oder müssen die Stege, der bzw. die die Stirnscheibe mit dem Stirnring verbinden, genügend weit außerhalb der Zapfenmitte verlaufen.

Im folgenden wird die Erfindung anhand eines in der Zeichnung dargestellten Ausführungsbeispieles näher erläutert; es zeigen:

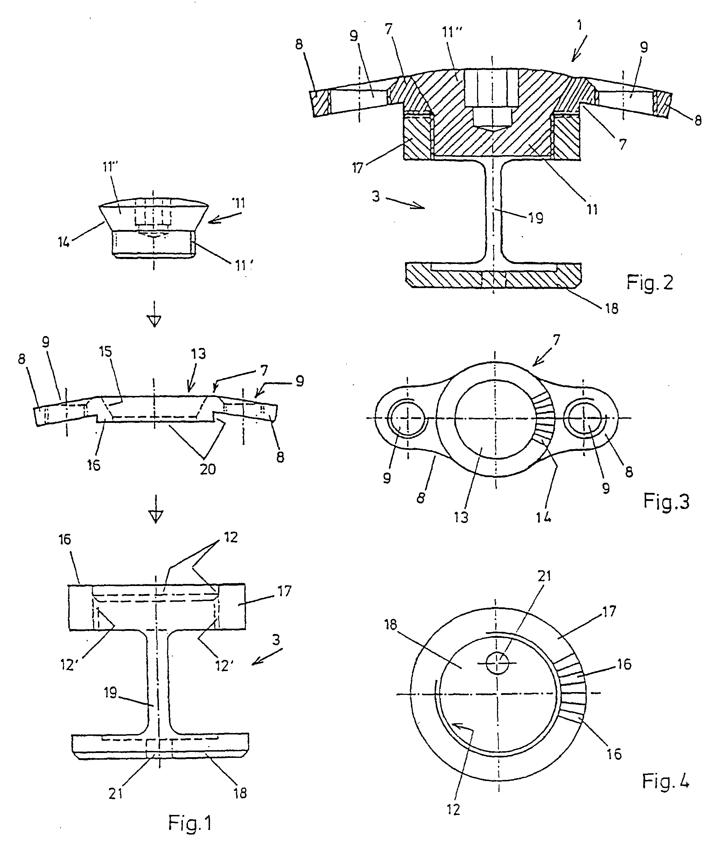

- Fig. 1: eine Seitenansicht des erfindungsgemäßen Implantats in einer Darstellung mit axial auseinander gezogenen Implantatteilen,
- Fig. 2: einen Axialschnitt durch das Implantat nach Fig. 1 im Zustand zusammengefügter Implantatteile,
- Fig. 3: eine axiale Draufsicht auf die Platte des Implantats, gesehen in Fig. 1 in Richtung von unten,
- Fig. 4: eine axiale Draufsicht auf den Zapfen des Implantats, gesehen in Fig. 1 in Richtung von oben,
- Fig. 5: den Schnitt in Richtung V - V in Fig. 4,
- Fig. 6: das Implantat im implantierten Zustand in einem Sagittalschnitt,
- Fig. 7: das Implantat nach Fig. 7 in einer Ansicht von vorn.

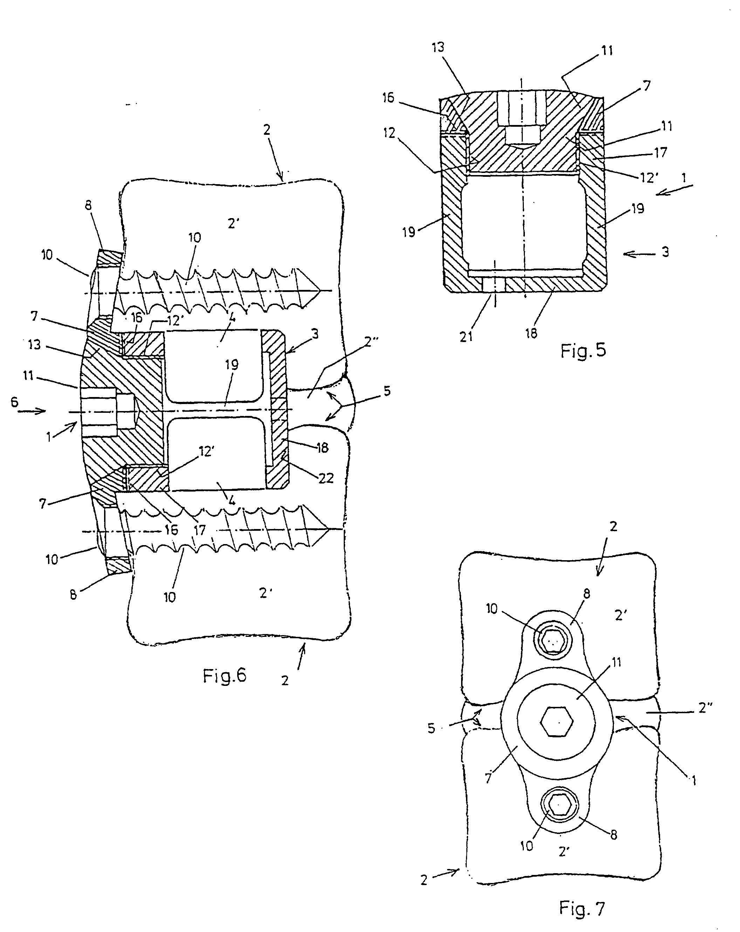

Das in der Zeichnung dargestellte Implantat 1 ist zum Fusionieren von zwei unmittelbar benachbarten Wirbeln 2 der Halswirbelsäule bestimmt. Es besitzt einen allgemein mit 3 bezeichneten hohlen Zapfen zum Einstecken in einen Bohrkanal 4, der an den sich gegenüber liegenden beiden Flächen 5 der Wirbelkörper 2' als Zapfenaufnahme ausgebildet ist. Der Zapfen 3 ist an seinem in der Einsteckrichtung, die in Fig. 6 durch den Pfeil 6 gekennzeichnet ist, rückwärtigen Ende lösbar an einer Platte 7 gehalten, die quer zur Einsteckrichtung beiderseits über den Zapfen 3 in Form je einer Zunge vorsteht und mit diesen zungenförmig vorstehenden Plattenteilen 8 bei in die Aufnahme eingestecktem Zapfen 3 die beiden benachbarten Wirbelkörper 2' übergreift, wie dies aus den Fig. 6 und 7 ersichtlich ist, wobei in Fig. 6 der Zapfenhohlraum zur besseren Deutlichkeit ohne Füllung mit Knochenzement oder autologem Knochenmaterial dargestellt ist. Die Platte 7 ist in den die Wirbelkörper 2' übergreifenden Plattenteilen 8 mit Löchern 9 für Knochenschrauben 10 versehen, mit welchen die Platte 7 an den beiden Wirbelkörpern 2' fixiert werden kann. Zur lösbaren Halterung des Zapfens 3 an der Platte 7 ist eine Verbindungsschraube 11 vorgesehen, die in koaxiale Öffnungen 12, 13 des Zapfens 3 und der Platte 7 einsetzbar ist. Dabei kann die Verbindungsschraube 11 mit ihrem Schraubengewinde 11' in ein an der Innenwand der Zapfenöffnung 12 ausgebildetes Muttergewinde 12' eingedreht werden. Wird die Verbindungsschraube 11 in diesem Muttergewinde 12' festgezogen, wird die Platte 7 gegen das sie abstützende Ende des Zapfens 3 verspannt. Der Schraubenkopf 11" der Verbindungsschraube 11 ist in die Öffnung 13 der Platte 7 eingesenkt, so daß er mit der äußeren Oberfläche der Platte 7 bündig abschließt. Im einzelnen ist der Schraubenkopf 11" mit einer kegelstumpfförmigen Ringfläche 14 versehen, mit welcher er einer entsprechenden hohlkegelförmigen Ringfläche 15 an der Innenwand der Plattenöffnung 13 aufsitzt, wenn die Verbindungsschraube 11 angezogen wird. Durch diesen Sitz der Verbindungsschraube 11 an der Platte 7 wird die Platte 7 durch die Verbindungsschraube 11 am Zapfen 3 zentriert. Die kegelstumpfförmige Ringfläche 14 am Schraubenkopf 11" erstreckt sich über die gesamte axiale Höhe des Schraubenkopfes, um eine möglichst große Sitzfläche zwischen der Verbindungsschraube 11 und der Platte 7 zu erhalten. Im übrigen sind am Zapfen 3 und an der Platte 7 einander zugeordnete Verzahnungen 16 ausgebildet, die bei angezogener Verbindungsschraube 11, also fest mit der Platte 7 verspanntem Zapfen 3, miteinander zum Eingriff kommen und ein gegenseitiges Verdrehen von Zapfen 3 und Platte 7 um die Achse der Verbindungsschraube 11 verhindern. Die Verzahnungen 16 sind als Ringverzahnungen ausgebildet, welche die Öffnungen 12, 13 in der Platte 3 bzw. im Zapfen 7 umgeben und mit radial zur Achse der Verbindungsschraube 11 gerichteten Zahnflächen ausgebildet sind.

Der Zapfen 3 ist im Ausführungsbeispiel, da das Implantat zur Verwendung im Bereich der Halswirbelsäule bestimmt ist, als überwiegend hohler Körper ausgebildet. Dieser Zapfenkörper besitzt am plattenseitigen Zapfenende einen mit der Platte 7 verbindbaren Stirnring 17 und am anderen Zapfenende eine Stirnscheibe 18 sowie zwei sich diametral zur Zapfenachse gegenüberstehende, den Stirnring 17 mit der Stirnscheibe 18 verbindende Stege 19. Die Ebenen des Stirnrings 17 und der Stirnscheibe 18 sind senkrecht und die sie verbindenden Stege 19 parallel zur Einsteckrichtung des Zapfens 3 in den Bohrkanal 4 ausgerichtet. Der Stirnring 17 und die Stirnscheibe 18 besitzen kreisförmigen Umriß bei gleichem Kreisdurchmesser, wobei die Stege 19 innerhalb dieses kreisförmigen Umrisses liegen. Der Stirnring 17 ist an seiner inneren Umfangswandung mit dem Muttergewinde 12' für die Verbindungsschraube 11 versehen. Die Platte 7 besitzt einen ihre Öffnung 13 für die Verbindungsschraube 11 umgebenden Kragen 20, an dem sich der stirnring 17 des Zapfens 3 abstützt. Der Kragen 20 und der Zapfen 3 stimmen in ihrem äußeren Umriß und Durchmesser überein und tragen an ihren einander zugekehrten Ringflächen die Ringverzahnungen 16. Im übrigen ist die Stirnscheibe 18 mit einer Aufnahme 21 zum Einsetzen eines selbst nicht dargestellten Instrumentes versehen, mit dessen Hilfe der Zapfen 3 bei zunächst noch fehlender Platte 7 in den Bohrkanal 4 eingesteckt und darin kontrolliert positioniert werden kann. Damit zu diesem Zweck das Instrument durch die hohle Mitte des Zapfens 3 hindurch in die Aufnahme eingeführt werden kann, müssen die Stege 19 ausreichend weit außerhalb der Zapfenachse verlaufen.

Die Implantation des beschriebenen Implantats 1 erfolgt von vorn, wie dies die Fig. 6 erkennen läßt. Dazu wird, nachdem die Bandscheibe im Wirbelzwischenraum 2" teilweise oder vollständig entfernt worden ist, an den einander gegenüber liegenden Flächen der Wirbelkörper 2' der Bohrkanal 4 für den Zapfen 3 ausgebildet, wobei der Bohrkanal an seinem in Einsteckrichtung des Zapfens vorderen Ende 22 blind im Wirbelkörper 2' endet. Der Zapfen 3 wird zunächst für sich, also gelöst von der Platte 7, in den Bohrkanal 4 eingetrieben. Hat der Zapfen 3 den bezüglich Tiefe und Drehlage gewünschten Sitz im Bohrkanal 4 eingenommen, wird die Platte 7 gegen den Zapfen 3 und die Wirbelkörper 2' gesetzt, mit dem Zapfen 3 verbunden und durch die Knochenschrauben 10 mit den beiden Wirbelkörpern 2' verschraubt.

## Patentansprüche

1. Implantat (1) zum Fusionieren von zwei unmittelbar benachbarten Wirbeln (2) der Wirbelsäule, mit einem Zapfen (3) zum Einstecken in einen an den sich gegenüber liegenden beiden Flächen der Wirbelkörper (2') als Zapfenaufnahme ausgebildeten Bohrkanal (4), wobei der Zapfen (3) an seinem in Einsteckrichtung rückwärtigen Ende lösbar an einer Platte (7) gehalten ist, die quer zur Einsteckrichtung (6) beiderseits über den Zapfen (3) vorsteht und mit den vorstehenden Plattenteilen (8) bei in die Aufnahme eingestecktem Zapfen (3) die beiden benachbarten Wirbelkörper (2') übergreift, sowie mit Löchern (9) für Knochenschrauben (10) versehen ist, mit welchen die Platte (7) an den beiden Wirbelkörpern (2') fixierbar ist, wobei zur lösbaren Halterung des Zapfens (3) an der Platte (7) eine Verbindungsschraube (11) vorgesehen ist, die in koaxiale Öffnungen (12, 13) des Zapfens (3) und der Platte (7) einsetzbar und dabei mit dem Schraubengewinde (11') in ein an der Innenwand der Zapfenöffnung (12) ausgebildetes Muttergewinde (12') eindrehbar ist, **dadurch gekennzeichnet, daß** am Zapfen (3) und an der Platte (7) einander zugeordnete Verzahnungen (16) ausgebildet sind, die bei an der Platte (7) gehaltenem Zapfen (3) miteinander zum Eingriff kommen und ein gegenseitiges Verdrehen von Zapfen (3) und Platte (7) um die Achse der Verbindungsschraube (11) verhindern, und daß die Verzahnungen (16) als die Öffnungen (12, 13) in der Platte (7) bzw. im Zapfen (3) umgebende Ringverzahnung mit radial zur Achse der Verbindungsschraube (11) gerichteten Zahnflächen ausgebildet sind.

2. Implantat nach Anspruch 1, **dadurch gekennzeichnet, daß** der Schraubenkopf (11") der Verbindungsschraube (11) in die Öffnung (13) der Platte (7) eingesenkt ist, wobei der Schraubenkopf (11") mit einer kegelstumpfförmigen Ringfläche (14) einer entsprechenden Ringfläche (15) an der Innenwand der Plattenöffnung (13) aufsitzt.

3. Implantat nach Anspruch 2, **dadurch gekennzeichnet, daß** sich die kegelstumpfförmige Ringfläche (14) am Schraubenkopf (11") über die gesamte axiale Höhe des Schraubenkopfes erstreckt.

4. Implantat nach einem der Ansprüche 1 bis 3, **dadurch gekennzeichnet, daß** der Zapfen (3) als überwiegend hohler Körper ausgebildet ist, der am plattenseitigen Zapfenende einen mit der Platte (7) verbindbaren Stirnring (17) und am anderen Zapfenende eine Stirnscheibe (18) sowie mindestens einen den Stirnring (17) mit der Stirnscheibe (18) verbindenden Steg (19) aufweist.

5. Implantat nach Anspruch 4, **dadurch gekennzeichnet, daß** die Ebenen des Stirnrings (17) und der Stirnscheibe (18) senkrecht und die sie verbindenden Stege (19) parallel zur Einsteckrichtung (6) des Zapfens (3) in den Bohrkanal (4) ausgerichtet sind.

6. Implantat nach Anspruch 4 oder 5, **dadurch gekennzeichnet, daß** der Stirnring (17) und die Stirnscheibe (18) kreisförmigen Umriß bei gleichem Kreisdurchmesser auf weisen.

7. Implantat nach einem der Ansprüche 4 bis 6, **dadurch gekennzeichnet, daß** der Stirnring (17) an seiner inneren Umfangswandung mit dem Muttergewinde (12') für die Verbindungsschraube (11) versehen ist.

8. Implantat nach einem der Ansprüche 4 bis 7, **dadurch gekennzeichnet, daß** die Platte (7) einen ihre Öffnung (13) für die Verbindungsschraube (11) umgebenden Kragen (20) aufweist, an dem sich der Stirnring (17) des Zapfens (3) abstützt.

9. Implantat nach Anspruch 8, **dadurch gekennzeichnet, daß** der Kragen (20) und der Zapfen (3) in ihrem äußeren Umriß übereinstimmen und an ihren einander zugeordneten Ringflächen die Verzahnungen (16) aufweisen.

10. Implantat nach einem der Ansprüche 4 bis 9, **dadurch gekennzeichnet, daß** die Stirnscheibe (18) eine Aufnahme (21) zum Einsetzen eines Instrumentes aufweist, mit dem der Zapfen (3) bei zunächst noch fehlender Platte (7) in den Bohrkanal (4) eingesteckt und darin kontrolliert positioniert werden kann.

## Claims

1. An implant (1) for fusing two immediately adjacent vertebrae (2) of the spinal column, comprising a peg (3) for insertion into a drilled passage (4) provided at the mutually opposite two surfaces of the vertebral bodies (2') as a peg-receiving means, wherein at its rearward end in the insertion direction the peg (3) is held releasably to a plate (7) which projects on both sides beyond the peg (3) transversely with respect to the insertion direction (6) and with the projecting plate portions (8) when the peg (3) is inserted Into the peg-receiving means engages over the two adjacent vertebral bodies (2'), and is provided with holes (9) for bone screws (10) with which the plate (7) is fixable to the two vertebral bodies (2'), wherein for releasably holding the peg (3) to the plate (7) there is provided a connecting screw (11) which can be inserted into coaxial openings (12, 13) in the peg (3) and the plate (7) and in that case can be screwed with the screw thread (11) into a female screwthread (12') provided at the inside wall of the peg opening (12), **characterised in that** provided on the peg (3) and on the plate (7) are mutually associated tooth configurations (16) which when the peg (3) is held to the plate (7) come into engagement with each other and prevent mutual rotation of the peg (3) and the plate (7) about the axis of the connecting screw (11) and that the tooth configurations (16) are in the form of an annular tooth configuration which extends around the openings (12, 13) in the plate (7) and in the peg (3) respectively and which has tooth faces directed radially with respect to the axis of the connecting screw (11).

2. An implant according to claim 1 **characterised in that** the screw head (11") of the connecting screw (11) is countersunk into the opening (13) in the plate (7), wherein the screw head (11") sits with a frustoconical annular surface (14) on a corresponding annular surface (15) at the inside wall of the plate opening (13).

3. An implant according to claim 2 **characterised in that** the frustoconical annular surface (14) on the screw head (11") extends over the entire axial height of the screw head.

4. An implant according to one of claims 1 to 3 **characterised in that** the peg (3) is in the form of a predominantly hollow body which at the peg end towards the plate has an end ring (17) connectable to the plate (7) and at the other peg end an end disc (18), and at least one bar (19) connecting the end ring (17) to the end disc (18).

5. An implant according to claim 4 **characterised in that** the planes of the end ring (17) and the end disc (18) are oriented perpendicularly and the bars (19) connecting them are oriented parallel to the insertion direction (6) of the peg (3) into the drilled passage (4).

6. An implant according to claim 4 or claim 5 **characterised in that** the end ring (17) and the end disc (18) have a circular contour with the same circle diameter.

7. An implant according to one of claims 4 to 6 **characterised in that** at its inner peripheral wall the end ring (17) is provided with a female screwthread (12') for the connecting screw (11).

8. An implant according to one of claims 4 to 7 **characterised in that** the plate (7) has a collar (20) which surrounds its opening (13) for the connecting screw (11) and against which the end ring (17) of the peg (3) bears.

9. An implant according to claim 8 **characterised in that** the collar (20) and the peg (3) are the same in their external contour and have the tooth configurations (16) at their mutually associated annular faces.

10. An implant according to one of claims 4 to 9 **characterised in that** the end disc (18) has a receiving means (21) for the insertion of an instrument with which the peg (3), when the plate (7) is initially still missing, can be inserted into the drilled passage (4) and controlledly positioned therein.

## Revendications

1. Implant (1) pour fusionner deux vertèbres (2) directement adjacentes de la colonne vertébrale, comprenant un tenon (3) destiné à être inséré dans un canal (4) foré en tant que logement pour le tenon dans les deux surfaces en regard des corps de vertèbres (2'), le tenon étant fixé par son extrémité située en arrière dans le sens d'insertion de façon amovible à une plaque (7) qui dépasse le tenon (3) transversalement à la direction d'insertion sur deux côtés, dont les parties dépassantes (8) recouvrent les deux corps de vertèbres (2') adjacents lorsque le tenon (3) est inséré dans le logement et qui présente des trous (9) pour des vis (10) à l'aide desquelles la plaque (7) peut être fixée aux deux corps de vertèbres (2'), la fixation amovible du tenon (3) à la plaque (7) s'effectuant à l'aide d'une vis d'assemblage (11) qui peut être engagée dans des ouvertures (12, 13) coaxiales du tenon (3) et de la plaque (7), le filetage (11') de la vis pouvant être vissé dans un filetage femelle (12') pratiqué dans la paroi intérieure de l'ouverture (12) du tenon, **caractérisé par le fait que** le tenon (3) et la plaque (7) présentent des dentures (16) associées qui entrent en prise lorsque le tenon (3) est fixé à la plaque (7) et empêchent une rotation réciproque du tenon (3) et de la plaque (7) autour de l'axe de la vis d'assemblage (11), et que les dentures (16) sont réalisées sous forme de denture annulaire entourant les ouvertures (12, 13) dans la plaque (7) et dans le tenon (3) et comportant des faces de dents dirigées radialement par rapport à l'axe de la vis d'assemblage (11).

2. Implant suivant la revendication 1, **caractérisé par le fait que** la tête (11") de la vis d'assemblage (11) est noyée dans l'ouverture (13) de la plaque (7), une surface annulaire tronconique (14) de la tête de vis (11") portant contre une surface annulaire (15) correspondante de la paroi intérieure de l'ouverture (13) de la plaque.

3. Implant suivant la revendication 2, **caractérisé par le fait que** la surface annulaire tronconique (14) de la tête de vis (11") s'étend sur toute la hauteur axiale de la tête de vis.

4. Implant suivant l'une des revendications 1 à 3, **caractérisé par le fait que** le tenon (3) est réalisé sous forme de corps essentiellement creux présentant à son extrémité côté plaque un anneau frontal (17) pouvant être relié à la plaque (7) et à son autre extrémité un disque frontal (18), ainsi qu'au moins une entretoise (19) reliant l'anneau frontal (17) au disque frontal (18).

5. Implant suivant la revendication 4, **caractérisé par le fait que** les plans de l'anneau frontal (17) et du disque frontal (18) sont perpendiculaires à, et les entretoises qui les relient entre eux sont parallèles à, la direction d'insertion (6) du tenon (3) dans le canal (4) foré.

6. Implant suivant la revendication 4 ou 5, **caractérisé par le fait que** l'anneau frontal (17) et le disque frontal (18) présentent un contour circulaire de même diamètre.

7. Implant suivant l'une des revendications 4 à 6, **caractérisé par le fait que** l'anneau frontal (17) comporte dans sa paroi périphérique intérieure le filetage femelle (12') pour la vis d'assemblage (11).

8. Implant suivant l'une des revendications 4 à 7, **caractérisé par le fait que** la plaque (7) présente un col (20) qui entoure son ouverture (13) pour la vis d'assemblage (11) et contre lequel porte l'anneau frontal (17) du tenon (3).

9. Implant suivant la revendication 8, **caractérisé par le fait que** le col (20) et le tenon (3) présentent le même contour extérieur et comportent les dentures (16) sur leurs faces annulaires associées.

10. Implant suivant l'une des revendications 4 à 9, **caractérisé par le fait que** le disque frontal (18) présente un logement (21) pour l'engagement d'un instrument à l'aide duquel le tenon (3), sans la plaque (7), peut être inséré et positionné de façon contrôlée dans le canal (4) foré.
